(19) Europäisches Patentamt
European Patent Office
Office européen des brevets

(11) Veröffentlichungsnummer: **0 480 241 A1**

(12) # EUROPÄISCHE PATENTANMELDUNG

(21) Anmeldenummer: **91116291.5**

(22) Anmeldetag: **25.09.91**

(51) Int. Cl.[5]: **C07D 413/04**, C07D 271/04, A61K 31/495, //(C07D413/04, 271:00,241:00)

(30) Priorität: **04.10.90 DE 4031373**

(43) Veröffentlichungstag der Anmeldung: **15.04.92 Patentblatt 92/16**

(84) Benannte Vertragsstaaten: **AT BE CH DE DK ES FR GB GR IT LI NL SE**

(71) Anmelder: **CASSELLA Aktiengesellschaft**
**Hanauer Landstrasse 526**
**W-6000 Frankfurt am Main 60(DE)**

(72) Erfinder: **Schönafinger, Karl, Dr.**
**Holunderweg 8**
**W-8755 Alzenau(DE)**
Erfinder: **Bohn, Helmut, Dr.**
**Kranzbergring 11**
**W-6369 Schöneck 1(DE)**
Erfinder: **Just, Melitta, Dr.**
**Theodor-Heuss-Strasse 80**
**W-6070 Langen 2(DE)**

(74) Vertreter: **Urbach, Hans-Georg, Dr. et al**
**Hanauer Landstrasse 526**
**W-6000 Frankfurt am Main 61(DE)**

(54) **3-Piperazino-sydnonimine, Verfahren zu ihrer Herstellung und ihre Verwendung.**

(57) Die Erfindung betrifft pharmakologisch wirksame substituierte 3-Aminosydnonimine der allgemeinen Formel I

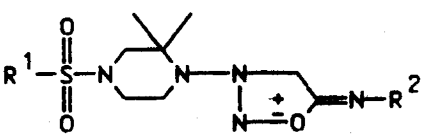

(I)

worin
$R^1$ $(C_1-C_4)$-Alkyl, Di-$(C_1-C_4)$-alkylamino oder $(C_6-C_{12})$-Aryl, das gegebenenfalls durch $(C_1-C_4)$-alkyl substituiert sein kann;
$R^2$ Wasserstoff oder $COR^3$ und
$R^3$ $(C_1-C_4)$-Alkyl, $(C_1-C_4)$-Alkoxy, $(C_1-C_4)$-Alkoxy-$(C_1-C_4)$-alkyl, Acetoxy$(C_1-C_4)$alkyl, $(C_6-C_{12})$-Aryl, das durch $(C_1-C_4)$-Alkyl oder $(C_1-C_4)$-Alkoxy substituiert sein kann, Pyridyl, Pyridyl$(C_1-C_4)$alkyl oder $(C_3-C_8)$-Cycloalkyl bedeuten
und ihre pharmakologisch annehmbaren Säureadditionssalze, ein Verfahren zu ihrer Herstellung und ihre Verwendung.

EP 0 480 241 A1

Die Erfindung betrifft pharmakologisch wirksame, substituierte 3-Aminosydnonimine der allgemeinen Formel I

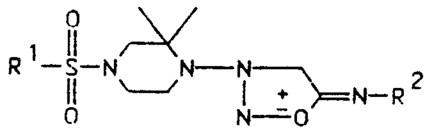

(I)

worin
$R^1$ $(C_1-C_4)$-Alkyl, Di-$(C_1-C_4)$-alkylamino oder $(C_6-C_{12})$-Aryl, das gegebenenfalls durch $(C_1-C_4)$-Alkyl substituiert sein kann;
$R^2$ Wasserstoff oder $COR^3$ und
$R^3$ $(C_1-C_4)$-Alkyl, $(C_1-C_4)$-Alkoxy, $(C_1-C_4)$-Alkoxy-$(C_1-C_4)$-alkyl, Acetoxy-$(C_1-C_4)$-alkyl, $(C_6-C_{12})$-Aryl, das durch $(C_1-C_4)$-Alkyl oder $(C_1-C_4)$-Alkoxy substituiert sein kann, Pyridyl, Pyridyl$(C_1-C_4)$alkyl oder $(C_3-C_8)$-Cycloalkyl bedeuten
und ihre pharmakologisch annehmbaren Säureadditionssalze.

Die Erfindung betrifft ferner ein Verfahren zur Herstellung der erfindungsgemäßen Verbindungen und ihre Verwendung.

Alkylreste, Alkoxyreste, Alkoxyalkylreste, Acetoxyalkylreste und Dialkylaminoreste können geradkettig oder verzweigt sein. Dies gilt auch, wenn sie als Substituenten für Aryl oder Pyridyl auftreten.

$(C_1-C_4)$Alkylreste können sein: Methyl, Ethyl, n-Propyl, i-Propyl, n-Butyl, i-Butyl, tert.-Butyl, sec.-Butyl.

Alkoxy-, Alkoxyalkyl-, Acetoxyalkyl-, Dialkylamino-, sowie Pyridylalkylreste leiten sich von den genannten Alkylresten ab.

$(C_3-C_8)$-Cycloalkylreste sind insbesondere Cyclopentyl und Cyclohexyl.

$(C_6-C_{12})$-Aryl ist insbesondere Phenyl, substituiertes $(C_6-C_{12})$-Aryl insbesondere p-Tolyl und p-Methoxyphenyl. Pyridyl ist insbesondere Pyridyl-(3), Pyridylalkyl insbesondere Pyridyl-(3)-alkyl.

$R^1$ bedeutet bevorzugt Methyl, p-Tolyl oder Dimethylamino.

$R^3$ bedeutet bevorzugt Ethyl, i-Propyl, tert-Butyl, Ethoxy, Pyridyl, p-Methoxyphenyl oder Cyclohexyl.

Eine Verbindung der allgemeinen Formel I kann dadurch hergestellt werden, daß eine Verbindung der allgemeinen Formel II

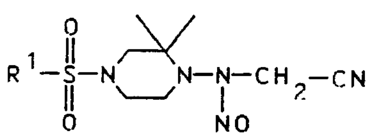

(II)

worin $R^1$ wie oben definiert ist, zu einer Verbindung der allgemeinen Formel Ia

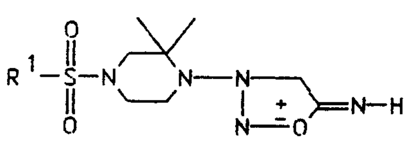

(Ia)

cyclisiert wird und daß diese oder ein Säureadditionssalz davon für den Fall, daß eine Verbindung der Formel I mit $R^2$ = $-COR^3$ hergestellt werden soll, mit einem Acylierungsmittel, das den Rest $-COR^3$ einführt, acyliert wird und die so erhaltene Verbindung gegebenenfalls in ein pharmakologisch annehmbares Säureadditionssalz überführt wird.

Die Cyclisierung der Verbindung II zu der Verbindung Ia wird in einem geeigneten organischen oder anorganischen Lösungs-, Dispergier- oder Verdünnungsmittel unter Zusatz eines Cyclisierungsmittels, normalerweise bei Temperaturen von -10 bis 40°C, insbesondere 0 bis 40°C, vorzugsweise bei 0 bis 20°C, durchgeführt.

Als Cyclisierungsmittel sind solche geeignet, die in wäßriger Lösung einen pH-Wert unter 3 einstellen, also z.B. Mineralsäuren, wie Schwefel-, Salpeter- oder Phosphorsäure, vorzugsweise Chlorwasserstoff, aber

auch starke organische Säuren, wie Trifluoressigsäure. Die Cyclisierung wird normalerweise unter Eiskühlung durchgeführt.

Von dem Cyclisierungsmittel kommen z.B. bezogen auf 1 mol der Verbindung der Formel II 0,1 bis 10 mol, vorzugsweise 1 bis 5 mol, zur Anwendung. Das Cyclisierungsmittel wird normalerweise im Überschuß eingesetzt. Besonders vorteilhaft ist die Verwendung von Chlorwasserstoff als Cyclisierungsmittel, der normalerweise in den Reaktionsansatz eingeleitet wird. Bei der Cyclisierung wird normalerweise das entsprechende Säureadditionssalz der Verbindung Ia erhalten.

Geeignete Lösungs-, Dispergier- oder Verdünnungsmittel sind z.B.: Alkohole, beispielsweise solche mit 1 bis 8 C-Atomen, insbesondere solche mit 1 bis 6 C-Atomen, vorzugsweise solche mit 1 bis 4 C-Atomen, wie z.B. Methanol, Ethanol, i- und n-Propanol, i-, sec- und tert-Butanol, n-, i-, sec-, tert-Pentanol, n-Hexanol, 2-Ethylbutanol, 2-Ethylhexanol, Isooctylalkohol, Cyclopentanol, Cyclohexanol, Methylcyclohexanol (Gemisch), Benzylalkohol; Ether, insbesondere solche mit 2 bis 8 C-Atomen im Molekül, wie z.B. Diethylether, Methyl-ethylether, Di-n-propyl-ether, Di-isopropyl-ether, Methyl-n-butylether, Methyl-tert-butylether, Ethyl-propyl-ether, Di-butylether, Tetrahydrofuran, 1,4-Dioxan, 1,2-Dimethoxyethan, Bis-$\beta$-methoxyethylether; Oligoethylen-glykol-dimethylether, wie z.B. Tetraglyme oder Pentaglyme; Carbonsäurealkylester, insbesondere solche mit 2 bis 10 C-Atomen im Molekül, wie z.B. Ameisensäure-methyl-, -ethyl-, -butyl- oder -isobutyl-ester, Essigsäure-methyl-, -ethyl-, -propyl-, isopropyl-, -butyl-, -isobutyl- oder -sec-butyl-, -amyl-, -isoamyl-, -hexyl-, -cyclohexyl- oder -benzyl-ester, Propionsäure-methyl-, -ethyl- oder -butyl-ester; Ketone, insbesondere solche mit 3 bis 10 C-Atomen im Molekül, wie z.B. Aceton, Methylethylketon, Methyl-n-propylketon, Diethylketon, 2-Hexanon, 3-Hexanon, Di-n-propylketon, Di-iso-propylketon, Di-iso-butylketon, Cyclopentanon, Cyclohexanon, Methylcyclohexanon, Dimethylcyclohexanon, Benzophenon, Acetophenon; aliphatische Kohlenwasserstoffe, wie z.B. Hexan, Heptan, niedrig-und hochsiedende Petrolether, Spezialbenzine und Testbenzin; cycloaliphatische Kohlenwasserstoffe, wie z.B. Cyclopentan, Cyclohexan, Methylcyclohexan, Tetralin, Decalin; aromatische Kohlenwasserstoffe, wie z.B. Benzol, Toluol, o-, m- und p-Xylol, Ethylbenzol; halogenierte aliphatische oder aromatische Kohlenwasserstoffe, wie z.B. Methylenchlorid, Chloroform, Tetrachlorkohlenstoff, 1,2-Dichlorethan, Chlorbenzol, Dichlorbenzol; Hexamethylphosphorsäuretriamid; Sulfoxide, wie z.B. Dimethyl-sulfoxid; Tetramethylensulfon; Wasser. Auch Gemische verschiedener Lösungs- oder Dispergiermittel können verwendet werden, beispielsweise Wasser-Methanol oder vorzugs weise Essigsäureethylester-Methanol.

Die Verbindung der Formel Ia stellt die erfindungsgemäße Verbindung der allgemeinen Formel I für den Fall dar, daß $R^2$ = Wasserstoff ist.

Die Acylierung der Verbindung der Formel Ia, die auch in Form eines Säureadditionssalzes vorliegan kann, zur Einführung des Restes $R^2$ = $-COR^3$ kann in an sich bekannter Weise mit einem geeigneten Acylierungsmittel der Formel III durchgeführt werden

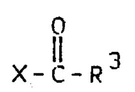

(III)

worin X ein nukleophil abspaltbarer Rest darstellt.

In der Formel III bedeutet X z.B. insbesondere Halogen, vorzugsweise -Cl oder -Br; -OH; -O-Alkyl, insbesondere mit 1 bis 5 C-Atomen; -O-Aryl, wobei der Arylrest insbesondere ein Phenylrest ist, der auch mit Alkyl, insbesondere Methyl, und/oder Nitro ein- oder mehrfach substituiert sein kann und beispielsweise ein Tolyl-, Dinitrophenyl- oder Nitrophenyl-Rest ist; $-O-CO-R^3$; -O-CO-O-Alkyl, insbesondere mit 1 bis 5 C-Atomen im Alkylrest, oder den über ein N-Atom gebundenen Rest eines Azols oder Benzazols mit mindestens 2 N-Atomen im quasi-aromatischen Fünfring.

Die Acylierung wird zweckmäßigerweise in flüssiger oder flüssiger disperser Phase in Anwesenheit eines inerten Lösungs-, Dispergier- oder Verdünnungsmittels oder in einem Überschuß des Acylierungsmittels, zweckmäßigerweise unter Rühren, durchgeführt.

Bei der Acylierung beträgt das Molverhältnis zwischen der Verbindung der Formel Ia und dem Acylierungsmittel der Formel III theoretisch 1 : 1. Das Acylierungsmittel kann jedoch auch im Über- oder Unterschuß eingesetzt werden. Zweckmäßigerweise wird das Acylierungsmittel der Formel III im Überschuß eingesetzt. Überschüsse von bis zu 50 mol% sind in der Regel ausreichend, d.h. das Molverhältnis zwischen der Verbindung der Formel Ia und dem Acylierungsmittel der Formel III beträgt normalerweise 1 : (1 bis 1,5), vorzugsweise 1: (1 bis 1,2). Falls bei der Acylierungsreaktion eine Säure abgespalten wird, ist der Zusatz eines Säurefängers, wie z.B. eines Alkylihydroxids, wie z.B. Natrium-, Kalium-oder Lithiumhydroxid, eines tertiären organischen Amins, wie z.B. Pyridin oder Triethylamin, eines Alkalicarbonats oder

Alkalibicarbonats, wie z.B. Soda oder Natriumbicarbonat, oder eines Alkalisalzes einer schwachen organischen Säure, wie z.B. Natriumacetat, zweckmäßig. Bei der Acylierungsreaktion können auch geeignete Katalysatoren, wie z.B. 4-Dimethylaminopyridin, zugesetzt werden.

Die Acylierung kann prinzipiell bei Temperaturen zwischen -10°C und dem Siedepunkt des verwendeten Lösungs-, Dispergier- oder Verdünnungsmittels erfolgen. In vielen Fällen wird die Umsetzung bei 0 bis 50°C, insbesondere bei 0 bis 30°C und vorzugsweise bei Raumtemperatur, durchgeführt.

Die Verbindungen der Formel III sind Acylierungsmittel und stellen somit z.B. dar: für X = Halogen: Säurehalogenide bzw. Halogenameisensäureester, von denen Säurechloride und Chlorameisensäureester bevorzugt sind; für -OH: Carbonsäuren; für -O-Alkyl und -O-Aryl: Ester, von denen die Tolyl-, 2,4-Dinitro- oder 4-Nitrophenylester bevorzugt sind; für -O-CO-$R^3$: Anhydride; für -O-CO-OAlkyl: gemischte Carbonsäure-Kohlensäure-Anhydride; oder heterocyclische Amide oder Azolide, insbesondere von N,N'-Carbonyldiazolen, wie z.B. N,N'-Carbonyldiimidazol, 2,2'-Carbonyl-ditriazol(1,2,3), 1,1'-Carbonyl-ditriazol-(1,2,4), N,N'-Carbonyl-dipyrazol, 2,2'-Carbonyl-ditriazol (vgl. z.B. H.A. Staab, M. Lücking und F.H. Dürr, Chem. Ber. 95, (1962), 1275 ff, H. A. Staab und A. Mannschreck, Chem. Ber. 95, (1962), 1284 ff.; H.A. Staab und W. Rohr, "Synthesen mit heterocyclischen Amiden (Azoliden)" in "Neuere Methoden der Präparativen Organischen Chemie", Band V, Verlag Chemie, 1967, S. 53 ff., insbesondere S. 65 bis 69). Die Acylierungsmittel der Formel III können nach an sich bekannten Verfahren hergestellt werden.

Bei der Verwendung einer Carbonsäure als Acylierungsmittel ist der Zusatz eines Aktivierungsmittels zweckmäßig, das die Aufgabe hat, das Acylierungspotential der Carbonsäure zu erhöhen oder zu aktivieren bzw. die Carbonsäure in situ oder vorzugsweise kurz vor der Umsetzung mit der Verbindung der Formel Ia in ein reaktives Carbonsäurederivat der Formel III zu überführen. Als derartige Aktivierungsmittel sind z.B. geeignet: N,N'-disubstituierte Carbodiimide, insbesondere wenn sie mindestens einen sekundären oder tertiären Alkylrest enthalten, wie z.B. Diisopropyl-, Dicyclohexyl- oder N-Methyl-N'-tert.butylcarbodiimid (vgl. Methodicum Chimicum, Verlag G.Thieme, Stuttgart, Bd.6, (1974), S.682/683, und Houben-Weyl, Methoden der Org. Chemie, Bd.8, (1952), S. 521/522); Kohlensäurederivate, wie z.B. Phosgen, Chlorameisensäureester, insbesondere mit 1 bis 5 C-Atomen im Alkylrest (vgl. z.B. Tetrahedron Letters 24 (1983), 3365 bis 3368); Kohlensäureester, wie z.B. N,N'-Disuccinimido-carbonat, Diphthalimido-carbonat, 1,1'-(Carbonyldioxy)-dibenzo-triazol oder Di-2-pyridyl-carbonat (vgl. z.B. Tetrahedron Letters, Vol.25, No.43, 4943-4946), gegebenenfalls in Anwesenheit geeigneter Katalysatoren, wie z.B. 4-Dimethylaminopyridin. Ferner sind als Aktivierungsmittel N,N'-Carbonyldiazole, wie z.B. N,N'-Carbonyl-diimidazol, 2,2'-Carbonyl-ditriazol(1,2,3), 1,1'-Carbonyl-ditriazol(1,2,4), N,N'-Carbonyl-dipyrazol, 2,2'-Carbonyl-ditetrazol, N,N'-Carbonyl-benzimidazol oder N,N'-Carbonylbenztriazol, geeignet (vgl. z.B. H.A. Staab, M. Lücking und F.H. Dürr, loc. cit; H.A. Staab und A. Mannschreck loc. cit.; H.A. Staab und W. Rohr loc. cit). Als N,N'-Carbonyldiazol wird häufig das käufliche N,N'-Carbonyl-diimidazol verwendet. Die anderen N,N'-Carbonylazole sind aber aus dem jeweiligen Azol und Phosgen ebenfalls leicht zugänglich.

Als Aktivierungsmittel für Carbonsäuren sind ferner geeignet: Derivate der Oxalsäure, wie z.B. Oxalylchlorid (vgl. z.B. GB-PS 2 139 225) oder N,N'-Oxalyl-diazole wie z.B. 1,1'-Oxalyldi-imidazol, 1,1'-Oxalyldi-1,2,4-triazol und 1,1'-Oxalyldi-1,2,3,4-tetrazol (vgl. z.B. Shizuaka Murata, Bull.Chem. Soc.Jap. 57, 3597-3598 (1984)); Methylethylphosphinsäureanhydrid (vgl. z.B. DE-OS 31 01 427); Diphosphortetrajodid (Chem. Lett. 1983, 449); Dialkyldisulfit (Indian J. Chem. 21, 259 (1982)); oder andere reaktive Agentien.

Geeignete Lösungs-, Dispergier- oder Verdünnungsmittel sind z.B. solche, die für die Durchführung der Cyclisierung angegeben worden sind, darüber hinaus auch z.B. Pyridin und Amide, wie z.B. Dimethylformamid. Neben Wasser werden für die Acylierung polare organische Lösungsmittel, wie Dimethylformamid, Dimethylsulfoxid oder Pyridin bevorzugt. Auch Lösungsmittelgemische, wie z.B. ein Gemisch aus Wasser und Methylenchlorid, sind geeignet.

Die Verbindungen der allgemeinen Formel I können mit anorganischen oder organischen Säuren Säureadditionssalze bilden. Geeignete Säuren für die Bildung pharmakologisch annehmbarer Säureadditionssalze sind beispielsweise: Chlorwasserstoff, Bromwasserstoff, Naphthalindisulfonsäuren, insbesondere Naphthalindisulfonsäure(1,5), Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion, Glucon-, Ascorbin-, Isonicotin-, Methansulfon-, p-Toluolsulfon-, Zitronen- oder Adipinsäure. Die Säureadditionssalze können wie üblich durch Vereinigung der Komponenten, zweckmäßigerweise in einem geeigneten Lösungs- oder Verdünnungsmittel, hergestellt werden.

Bei der Synthese der Verbindungen der allgemeinen Formel Ia fallen normalerweise die Säureadditionssalze an.

Die Ausgangsverbindung der allgemeinen Formel II kann in einfacher und an sich bekannter Weise nach der Streckerschen Aminonitrilsynthese aus Verbindungen der allgemeinen Formel IV

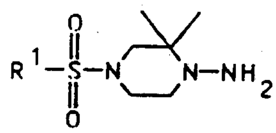

(IV)

worin $R^1$ wie oben definiert ist, durch Umsetzung mit Formaldehyd und Blausäure bzw. Natriumcyanid in einem geeigneten Lösungsmittel, z.B. Wasser, hergestellt werden, wobei zunächst eine Verbindung der allgemeinen Formel V

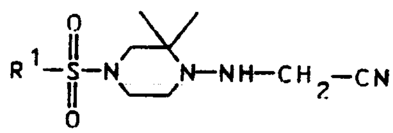

(V)

entsteht, die durch Nitrosierung in die Verbindung II überführt wird. Die Nitrosierung wird in bekannter Weise in einem geeigneten Lösungsmittel, vorzugsweise in Wasser, z.B. bei Temperaturen von 0 bis 10°C durchgeführt. Die salpetrige Säure wird dabei normalerweise aus einem Alkalimetallnitrit, z.B. Natriumnitrit, und Salzsäure erzeugt. Es ist zweckmäßig, die wäßrige Lösung der Verbindung V mit Salzsäure auf einen pH-Wert von 1 bis 3 einzustellen und das Alkalimetallnitrit in Form einer wäßrigen Lösung zu der gerührten und abgekühlten Lösung der Verbindung zuzutropfen.

Die Lösung der dabei erhaltenen Verbindung II kann direkt der Cyclisierungsreaktion unterworfen werden. Normalerweise ist es jedoch angebracht, die Nitrosoverbindung II zunächst in einem geeigneten organischen Lösungsmittel aufzunehmen und in ihm, gegebenenfalls nach Zusatz eines weiteren Lösungsmittels, die Cyclisierung zu der Verbindung der Formel Ia durchzuführen.

Die Verbindungen der allgemeinen Formel IV lassen sich, ausgehend von Verbindungen der allgemeinen Formel VI

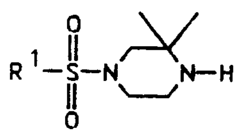

(VI)

dadurch herstellen, daß entweder

a) eine Verbindung der allgemeinen Formel VI zur N-Nitrosoverbindung VII nitrosiert und anschließend reduziert wird.

(VI)

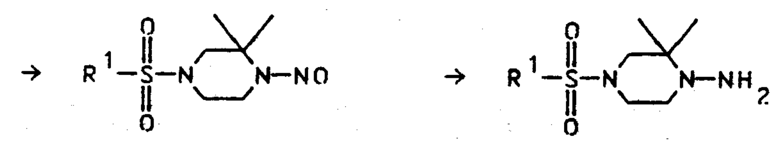

(VII) (IV)

oder daß in an sich bekannter Weise

b) eine Verbindung der allgemeinen Formel VI mit einem Alkalicyanat im sauren Medium in das Harnstoffderivat VIII überführt wird, das dann durch Hoffmann-Abbau zur Verbindung IV umgesetzt wird.

(VI)

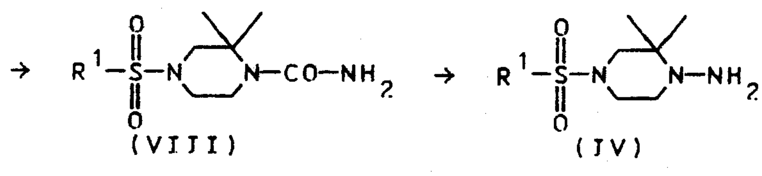

(VIII) (IV)

Die Nitrosierung der Verbindung der allgemeinen Formel VI zur Verbindung der allgemeinen Formel VII geschieht analog dem oben angegebenen Verfahren, die anschließende Reduktion wird in an sich bekannter Weise, bevorzugt mit Thioharnstoffdioxid duchgeführt.

Die Harnstoffsynthese durch Umsetzung eines Amins mit einem Alkalicyanat in saurer wäßriger Lösung (d.h. Umsetzung der Verbindung der allgemeinen Formel VI zur Verbindung der allgemeinen Formel VIII) ist bekannt und in der Literatur beschrieben (siehe z.B. Houben-Weyl, Methoden der Organischen Chemie, 4. Auflage Bd. E4, Seite 362). Ein bevorzugtes Alkalicyanat ist dabei Kaliumcyanat. Auch der Hofmann-Abbau, d.h. die Synthese eines Amins aus dem entsprechenden Amid durch Umsetzung mit Hypochlorit oder -bromit, ist literaturbekannt (siehe z.B. Houben-Weyl, Methoden der Organischen Chemie, 4. Auflage Bd. XI/1 Seiten 854 ff.).

Die Herstellung der Verbindungen der allgemeinen Formel VI erfolgt in an sich bekannter Weise durch Umsetzung des bekannten 2,2-Dimethylpiperazins mit den bekannten Sulfonylchloriden $R^1$-$SO_2$-Cl.

Die Verbindungen der allgemeinen Formel I und ihre pharmakologisch annehmbaren Säureadditionssalze besitzen wertvolle pharmakologische Eigenschaften. Besonders ausgeprägt ist ihre Wirkung auf das Herz-Kreislauf-System. Verglichen mit bekannten, in 3-Stellung substituierten Sydnoniminverbindungen z.B. solchen der EP-B-59356, sowie der im Handel befindlichen strukturähnlichen Verbindung Molsidomin, besitzen sie überraschenderweise eine stärkere und längere Wirkungsdauer. Sie senken beispielsweise den Blutdruck ebenso wie den Pulmonalarteriendruck und den linksventrikulären enddiastolischen Druck und tragen so zu einer Entlastung der Herztätigkeit im Sinne einer antianginösen Wirkung bei, ohne dabei eine reflektorische Tachykardie zu provozieren.

Durch Hemmung der Thrombocytenaggregation können die Verbindungen außerdem antithrombotische Effekte zeigen.

Die Verbindungen der Formel I und ihre pharmakologisch annehmbaren Säureadditionssalze können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I oder eines Säureadditionssalzes davon, neben üblichen pharmazeutisch einwandfreien Träger- und Zusatzstoffen enthalten.

Die Heilmittel können oral, z.B. in Form von Pillen, Tabletten, Lacktabletten, Dragees, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Zur Herstellung der pharmazeutischen Präparate können pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eig nen sich z.B. Wasser, Alkohole, Glyzerin, Polyole oder pflanzliche Öle.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der Formel I oder ihrer pharmakologisch annehmbaren Säureadditionssalze und noch andere therapeutisch wirksame Stoffe enthalten.

Derartige andere therapeutisch wirksame Substanzen sind beispielsweise: $\beta$-Rezeptorenblocker, wie z.B. Propranolol, Pindolol, Metoprolol; Vasodilatatoren, wie z.B. Carbochromen; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Ramipril, Prazosin, Clonidin, Rauwolfia-Alkaloide; Mittel, die den Fettsäurespiegel im Blut senken, wie z.B. Bezafibrat, Fenofibrat; Mittel für die Thromboseprophylaxe, wie z.B. Phenprocoumon.

Die Verbindungen der Formel I, ihre pharmakologisch annehmbaren Säureadditionsssalze und pharmazeutische Präparate, welche die Verbindungen der Formel I oder ihre pharmakologisch annehmbaren Säureadditionssalze als Wirkstoffe enthalten, können am Menschen bei der Bekämpfung bzw. Vorbeugung von Erkrankungen des kardiovaskulären Systems verwendet werden, beispielsweise als antihypertensive Heilmittel bei den verschiedenen Formen des Bluthochdrucks, bei der Bekämpfung bzw. Vorbeugung von

Angina pectoris usw. Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 0,5 bis 100 mg, vorzugsweise 1 bis 20 mg, angemessen. Auch bei anderen Applikationsformen liegt die Tagesdosis, wegen der guten Resorption der Wirkstoffe, in ähnlichen Mengenbereichen, d.h. im allgemeinen ebenfalls bei 0,5 bis 100 mg/Mensch. Die Tagesdosis wird normalerweise in mehrere, z.B. 2 bis 4 Teilverabreichungen aufgeteilt.

Die pharmakologische Wirkung der verbindungen der Formel I wurde nach einer modifizierten Methode von Godfraind and Kaba (Arch.Int. Pharmacodyn. Ther. 196, (Suppl) 35 bis 49, 1972) und von Schüman et al (Naunyn-Schmiedeberg's Arch. Pharmacol. 289, 409 bis 418, 1975) ermittelt. Dabei werden Spiralstreifen der Arteria pulmonalis des Meerschweinchens nach Äquilibrierung in calciumfreier Tyrodelösung mit 40 mmol/l Kalium depolarisiert. Ein Zusatz von 0,5 mmol/l $CaCl_2$ löst dann eine Kontraktion aus.

Die relaxierende Wirkung der Prüfsubstanz wird durch kumulative Zugabe in 1/2 log 10 abgestuften Konzentrationen ermittelt. Aus der Konzentrationswirkungskurve (Abszisse: -log mol/l Prüfsubstanz, Ordinate: % Hemmung der maximalen Kontraktion, Mittelwert von 4 bis 6 Gefäßstreifen) wird die Konzentration der Prüfsubstanz ermittelt, welche die Kontraktion um 50 % hemmt (= $IC_{50}$, mol/l). Die Wirkungsdauer der Prüfsubstanz ergibt sich aus der Zeit, die nach der Zugabe der Prüfsubstanz benötigt wird, bis der Ausganswert wieder erreicht ist. In der folgenden Tabelle sind die so erhaltenen Werte angegeben. Wie der Vergleich mit dem $IC_{50}$- Werte für die bekannte Verbindung Molsidomin (vgl. DE-B-16 95 897) ergibt, liegen die Werte für die Verbindungen der allgemeinen Formel I erheblich günstiger. Andererseits wird die ähnlich wirksame Verbindung SIN-1 hinsichtlich Wirkdauer von den erfindungsgemäßen Verbindungen deutlich übertroffen.

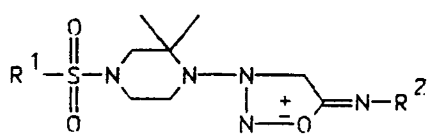

| $R^1$ | $R^2$ | $IC_{50}$ (mol/l) | Wirkdauer (min) |
|---|---|---|---|
| $-CH_3$ | -H | $1,9 \cdot 10^{-6}$ | 200 |
| 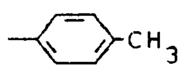 | -H | $6,5 \cdot 10^{-7}$ | 220 |
| $-N(CH_3)_2$ | -H | $2,5 \cdot 10^{-6}$ | 200 |
| $-CH_3$ | 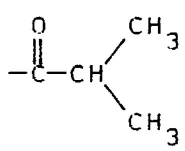 | $3 \cdot 10^{-6}$ | 210 |
| Molsidomin | | $3 \cdot 10^{-4}$ | 100 |
| $SIN^{-1}$ | | $1 \cdot 10^{-6}$ | 80 |

Molsidomin: N-Ethoxycarbonyl-3-morpholino-sydnonimin

SIN-1: 3-Morpholinosydnonimin · HCl

(Linsidomin)

BEISPIELE

1. 3-(2,2-Dimethyl-4-methansulfonyl-piperazin-1-yl)-sydnonimin-hydrochlorid

a) 4-Methansulfonyl-2,2-dimethyl-piperazin

Zur Mischung von 52,6 g 2,2-Dimethylpiperazin, 69,9 g Pottasche und 150 ml Toluol wird die Lösung von 52,8 g Methansulfonylchlorid in 100 ml Toluol getropft, wobei die Innentemperatur auf etwa 55°C ansteigt. Nach 3 Stunden Rührzeit wird der unlösliche Anteil abgesaugt und die Toluollösung im Vakuum eingedampft. Das verbleibende Öl erstarrt allmählich und wird zur Reinigung aus Isopropanol umkristallisiert.

Ausbeute: 51,2 g Fp. = 103-6°C

b) 4-Methansulfonyl-2,2-dimethyl-1-nitroso-piperazin

Die auf 0 - 5°C abgekühlte Mischung von 45 g 4-Methansulfonyl-2,2-dimethyl-piperazin, 19,3 g Natriumnitrit, 250 ml Wasser und 100 ml Methanol wird mit konz. HCl auf pH = 1,0 gestellt und bei allmählich ansteigender Temperatur 12 Stunden gerührt. Der Niederschlag wird abgesaugt, mit Wasser gewaschen und in Vakuum getrocket.

Ausbeute: 45 g Fp. 158 - 61°C

c) 1-Amino-4-methansulfonyl-2,2-dimethyl-piperazin

Die Mischung von 84,2 g 4-Methansulfonyl-2,2-dimethyl-1-nitroso-piperazin, 90,5 g Thioharnstoffdioxid, 200 ml Ethanol und 200 ml Wasser wird mit 85 g Ätznatron vorsichtig versetzt und 30 Stunden am Rückfluß erhitzt. Nach dem Abkühlen wird filtriert und aus dem Filtrat das Produkt durch Extraktion mit Methylenchlorid isoliert. Nach dem Trocknen über Natriumsulfat und Einengen der Methylenchloridlösung wird das verbleibende Öl in wenig Ethanol gelöst und durch Zugabe von ethanolischer Salzsäure als Hydrochlorid gefällt.

Ausbeute: 44 g Fp. 206°C (Zers.)

d) 3-(2,2-Dimethyl-4-methansulfonyl-piperazin-1-yl)-sydnonimin-hydrochlorid

Die Lösung von 48 g 1-Amino-2,2-dimethyl-4-methansulfonyl-piperazin-hydrochlorid in 300 ml Wasser wird im Eisbad gekühlt, mit 12 g Natriumcyanid versetzt und durch Zutropfen von konz. Salzsäure auf ein pH von 7-7,5 eingestellt. Dann werden 19 g einer 39%igen Formalinlösung zugegeben und das pH der Mischung mit Sodalösung auf etwa 7 gehalten, wobei bei Raumtemperatur gerührt wird. Nach 2 Stunden wird erneut auf 0 - 5°C abgekühlt, 14 g Natriumnitrit zugegeben und durch Zutropfen von konz. Salzsäure sauer (pH = 2,5-3) gestellt. Nach 2 Stunden wird die Mischung mit 200 ml Essigester ausgeschüttelt und die org. Phase über $Na_2SO_4$ getrocknet.Dann werden 100 ml einer konz. ethanolischen Salzsäure zugesetzt und mehrere Stunden im Eisbad gerührt. Der Niederschlag wird abgesaugt, mit Essigester gewaschen und getrocknet.

Ausbeute: 44,5 g Fp. 83-5°C (Zers.)

Analog Beispiel 1 wurden durch Verwendung von Tosylchlorid, bzw. Dimethylaminosulfonylchlorid anstelle von Methansulfochlorid die Verbindungen der Beispiele 5 und 6 erhalten.

2) 3-(2,2-Dimethyl-4-tosyl-piperazin-1-yl)-sydnoniminhydrochlorid

Fp. 190 - 92°C (Zers.)

3) 3-(2,2-Dimethyl-4-dimethylaminosulfonyl-piperazin-1-yl)-sydnonimin-hydrochlorid

Fp. 177-8°C (Zers.)

4) N-Nicotinoyl-3-(2,2-dimethyl-4-methansulfonyl-piperazin-1-yl)-sydnonimin

Zu einer eisgekühlten Lösung von 1,85 g Nikotinsäurechlorid in 30 ml Pyridin werden 2 g 8-(2,2-Dimethyl-4-methan-sulfonyl-piperazin-1-yl)-sydnonimin-hydrochlorid gegeben. Das Gemisch wird anschließend bei Raumtemperatur 15 Stunden gerührt und im Eisbad abgekühlt. Der Niederschlag wird abgesaugt und aus Ethanol umkristallisiert.

Ausbeute: 1,4 g Fp. 233°C Zers.

5) N-Butoxyacetyl-3-(2,2-dimethyl-4-methansulfonyl-piperazin-1-yl)-sydnonimin

Zur eisgekühlten Lösung von 2 g 3-(2,2-Dimethyl-4-methansulfonyl-piperazin-1-yl)-sydnonimin HCl in 20 ml Wasser wird Natriumbicarbonat (1,3 g) gegeben und anschließend die Lösung von 1 g Butoxyacetylchlorid in 30 ml Methylenchlorid rasch zugetropft. Nach Beendigung der Gasentwicklung wird die organische Phase abgetrennt, getrocknet und in Vakuum eingedampft. Das verbleibende Öl wird aus tert. Butylmethylether umkristallisiert.

Ausbeute: 1,3 g Fp. 106-8°C

Analog lassen sich die folgenden Beispiele durch Verwendung entsprechender Säurechloride herstellen:

6) N-p-Anisoyl-3-(2,2-dimethyl-4-methansulfonyl-piperazin-1-yl)-sydnonimin

Fp. 210°C (Zers.)

7) N-Ethoxicarbonyl-3-(2,2-dimethyl-4-methansulfonyl-piperazin-1-yl)-sydnonimin

Fp. 182°C (Zers.)

8) N-Butoxycarbonyl-3-(2,2-dimethyl-4-methansulfonyl-piperazin-1-yl)-sydnonimin

Fp. 164 - 6°C

9) N-Isobutyroyl-3-(2,2-dimethyl-4-methansulfonyl-piperazin-1-yl)-sydnonimin

Fp. 93 - 6°C

10) N-Acetoxyacetyl-3-(2,2-dimethyl-4-methansulfonyl-piperazin-1-yl)-sydnonimin

Fp. 112 - 114°C

11) N-Ethoxycarbonyl-3-(2,2-dimethyl-4-tosyl-piperazin-1-yl)-sydnonimin

Fp. 157 - 8°C

12) N-Ethoxycarbonyl-3-(2,2-dimethyl-4-dimethylaminosulfonyl-piperazin-1-yl)-sydnonimin

Fp. 132 - 4°C

13) N-Cyclohexylcarbonyl-3-(2,2-dimethyl-4-methansulfonyl-piperazin-1-yl)-sydnonimin

Fp. des Hydrochlorids: 169°C (Zers.)

14) N-Pivaloyl-3-(2,2-dimethyl-4-methansulfonyl-piperazin-1-yl)-sydnonimin

Öl, Fp. des Hydrochlorids: 158 - 9°C

15) N-(Pyrid-3-yl-methyl-carbonyl)-3-(2,2-dimethyl-4-methansulfonyl-piperazin-1-yl)-sydnonimin

Die Mischung von 1,4 g Pyridyl-3-essigsäure und 30 ml Methylenchlorid wird unter Eiskühlung mit 1,4 ml Triethylamln und 1,25 ml Pivaloylchlorid versetzt und dann 30 min gerührt. Das so erhaltene Reaktionsgemisch wird zur eisgekühlten Lösung von 2,4 g 3-(2,2-Dimethyl-4-methansulfonyl-piperazin-1-yl)-sydnonimin-hydrochlorid und 2,5 g Natriumbicarbonat in 20 ml Wasser gegeben und die erhaltene Mischung wie in Beispiel 5 angegeben weiter behandelt. Das erhaltene Rohprodukt wird aus Wasser umkristallisiert.

Ausbeute: 1,5 g Fp. 73-4°C

**Patentansprüche**

**1.** 3-Aminosydnonimine der allgemeinen Formel I

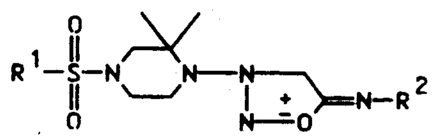

(I)

worin

$R^1$ $(C_1\text{-}C_4)$-Alkyl, Di-$(C_1\text{-}C_4)$-alkylamino oder $(C_6\text{-}C_{12})$-Aryl, das gegebenenfalls durch $(C_1\text{-}C_4)$-Alkyl substituiert sein kann;

$R^2$ Wasserstoff oder $COR^3$ und

$R^3$ $(C_1\text{-}C_4)$-Alkyl, $(C_1\text{-}C_4)$-Alkoxy, $(C_1\text{-}C_4)$-Alkoxy-$(C_1\text{-}C_4)$-alkyl, Acetoxy$(C_1\text{-}C_4)$alkyl, $(C_6\text{-}C_{12})$-Aryl, das durch $(C_1\text{-}C_4)$-Alkyl oder $(C_1\text{-}C_4)$-Alkoxy substituiert sein kann, Pyridyl, Pyridyl$(C_1\text{-}C_4)$alkyl oder $(C_3\text{-}C_8)$-Cycloalkyl bedeuten

und ihre pharmakologisch annehmbaren Säureadditionssalze.

**2.** 3-Aminosydnonimin gemäß Anspruch 1, dadurch gekennzeichnet, daß $R^1$ Methyl, p-Tolyl oder Dimethylamino bedeutet.

**3.** 3-Aminosydnonimin gemäß Anspruch 1 und/oder 2, dadurch gekennzeichnet, daß $R^3$ Ethyl, i-Propyl, tert-Butyl, Ethoxy, Pyridyl, p-Methoxyphenyl oder Cyclohexyl bedeutet.

**4.** Verfahren zur Herstellung eines 3-Aminosydnonimins der allgemeinen Formel I gemäß einem oder mehreren der Ansprüche 1 bis 3, dadurch gekennzeichnet, daß eine Verbindung der allgemeinen Formel II

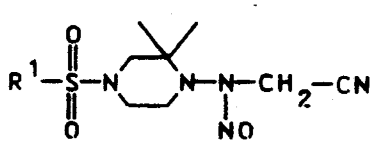

(II)

worin $R^1$ wie in Anspruch 1 definiert ist, zu einer Verbindung der allgemeinen Formel Ia

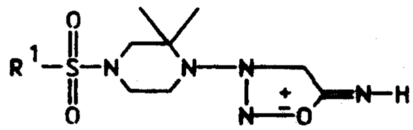

(Ia)

cyclisiert wird und daß diese oder ein Säureadditionssalz davon für den Fall, daß eine Verbindung der Formel I mit $R^2$ = $-COR^3$ hergestellt werden soll, mit einem Acylierungsmittel, das den Rest $-COR^3$ einführt, acyliert wird und die so erhaltene Verbindung gegebenenfalls in ein pharmakologisch annehmbares Säureadditionssalz überführt wird.

**5.** Verfahren nach Anspruch 4, dadurch gekennzeichnet, daß die Cyclisierung in einem Lösungs-, Dispergier- oder Verdünnungsmittel bei Temperaturen von -10 bis 40°C, vorzugsweise 0 bis 20°C, mit Hilfe eines Cyclisierungsmittels, das in wäßriger Lösung einen pH-Wert unter 3 einstellt, durchgeführt wird.

**6.** Verwendung von 3-Aminosydnoniminen der allgemeinen Formel I gemäß einem oder mehreren der Ansprüche 1 bis 3 oder pharmakologisch annehmbare Säureadditionssalze davon zur Herstellung von Arzneimitteln zur Bekämpfung und Vorbeugung cardiovaskulärer Erkrankungen.

**7.** Pharmazeutisches Präparat, dadurch gekennzeichnet, daß es ein 3-Aminosydnonimin der allgemeinen Formel I gemäß einem oder mehreren der Ansprüche 1 bis 3, oder ein pharmakologisch annehmbares Säureadditionssalz davon als Wirkstoff zusammen mit pharmazeutisch annehmbaren Träger- und Zusatzstoffen und gegebenenfalls noch ein oder mehrere andere pharmakologische Wirkstoffe enthält.

**Patentansprüche für folgende Vertragsstaaten : ES, GR**

**1.** Verfahren zur Herstellung von 3-Aminosydnoniminen der allgemeinen Formel I

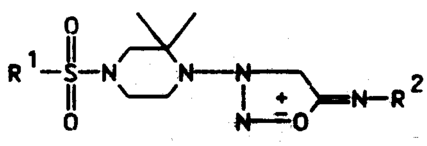

(I)

worin
$R^1$ $(C_1-C_4)$-Alkyl, Di-$(C_1-C_4)$-alkylamino oder $(C_6-C_{12})$-Aryl, das gegebenenfalls durch $(C_1-C_4)$-Alkyl substituiert sein kann;
$R^2$ Wasserstoff oder $COR^3$ und
$R^3$ $(C_1-C_4)$-Alkyl, $(C_1-C_4)$-Alkoxy, $(C_1-C_4)$-Alkoxy-$(C_1-C_4)$-alkyl, Acetoxy$(C_1-C_4)$alkyl, $(C_6-C_{12})$-Aryl, das durch $(C_1-C_4)$-Alkyl oder $(C_1-C_4)$-Alkoxy substituiert sein kann, Pyridyl, Pyridyl$(C_1-C_4)$alkyl oder $(C_3-C_8)$-Cycloalkyl bedeuten
und ihrer pharmakologisch annehmbaren Säureadditionssalze, dadurch gekennzeichnet, daß eine Verbindung der allgemeinen Formel II

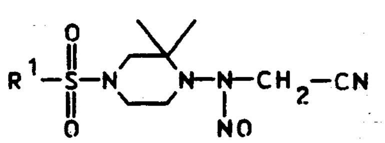

(II)

worin $R^1$ wie oben definiert ist, zu einer Verbindung der allgemeinen Formel Ia

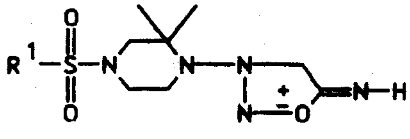

(Ia)

cyclisiert wird und daß diese oder ein Säureadditionssalz davon für den Fall, daß eine Verbindung der Formel I mit $R^2$ = $-COR^3$ hergestellt werden soll, mit einem Acylierungsmittel, das den Rest $-COR^3$ einführt, acyliert wird und die so erhaltene Verbindung gegebenenfalls in ein pharmakologisch annehmbares Säureadditionssalz überführt wird.

**2.** Verfahren nach Anspruch 1, dadurch gekennzeichnet, daß die Cyclisierung in einem Lösungs-, Dispergier- oder Verdünnungsmittel bei Temperaturen von -10 bis 40°C, vorzugsweise 0 bis 20°C, mit Hilfe eines Cyclisierungsmittels, das in wäßriger Lösung einen pH-Wert unter 3 einstellt, durchgeführt wird.

**3.** Verfahren gemäß Anspruch 1 und/oder 2, dadurch gekennzeichnet, daß in der allgemeinen Formel I $R^1$ Methyl, p-Tolyl oder Dimethylamino bedeutet.

**4.** Verfahren gemäß einem oder mehreren der Ansprüche 1 bis 3, dadurch gekennzeichnet, daß in der allgemeinen Formel I $R^3$ Ethyl, i-Propyl, tert-Butyl, Ethoxy, Pyridyl, p-Methoxyphenyl oder Cyclohexyl bedeutet.

**5.** Verwendung von 3-Aminosydnoniminen der allgemeinen Formel I gemäß einem oder mehreren der Ansprüche 1 bis 4 oder pharmakologisch annehmbare Säureadditionssalze davon zur Herstellung von Arzneimitteln zur Bekämpfung und Vorbeugung cardiovaskulärer Erkrankungen.

**6.** Verfahren zur Herstellung eines pharmazeutischen Präparates enthaltend ein 3-Aminosydnonimin der allgemeinen Formel I gemäß einem oder mehreren der Ansprüche 1 bis 4 oder ein pharmakologisch annehmbares Säureadditionssalz davon, dadurch gekennzeichnet, daß man diese zusammen mit pharmazeutisch annehmbaren Träger- und Zusatzstoffen und gegebenenfalls noch einem oder mehreren anderen phar-makologischen Wirkstoffen in eine geeignete Darreichungsform bringt.

Europäisches Patentamt

# EUROPÄISCHER RECHERCHENBERICHT

Nummer der Anmeldung

**EP 91 11 6291**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | EP-A-0 346 694 (CASSELLA AG)<br>* Ansprüche 1-5, 9-12 *<br>– – – | 1-7 | C 07 D 413/04<br>C 07 D 271/04<br>A 61 K 31/495 //<br>(C 07 D 413/04<br>C 07 D<br>C 07 D 271:00<br>C 07 D 241:00 ) |
| Y | EP-A-0 023 343 (CASSELLA AG)<br>* Ansprüche 1-5, 7, 11-13 *<br>* Beispiele 4, 5 *<br>– – – | 1-7 | |
| Y | EP-A-0 210 474 (CASSELLA AG)<br>* Ansprüche 1-3, 6, 9, 10 *<br>– – – | 1,2,4,6,7 | |
| A,D | EP-A-0 059 356 (CASSELLA AG)<br>* Ansprüche 1, 8, 11 *<br>– – – | 1,4,7 | |
| A,D | DE-A-1 695 897 (TAKEDA CHEMICAL INDUSTRIES, LTD.)<br>* Ansprüche 1, 2 *<br>* Beispiel 25 *<br>– – – – – | 1,7 | |

RECHERCHIERTE SACHGEBIETE (Int. Cl.5)

C 07 D 271/00
C 07 D 295/00
C 07 D 413/00

Der vorliegende Recherchenbericht wurde für alle Patentansprüche erstellt

| Recherchenort | Abschlußdatum der Recherche | Prüfer |
|---|---|---|
| Berlin | 02 Dezember 91 | HASS C V F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

&: Mitglied der gleichen Patentfamilie, übereinstimmendes Dokument